# EUROPEAN PATENT APPLICATION

(11) **EP 4 768 037 A1**
(43) Date of publication of application: **01.07.2026**
(21) Application number: 25164963.8
(22) Date of filing: 20.03.2025
(51) Int. Cl.: A61K 36/068, A61K 8/9728, A61P 17/16, A61P 17/18, A61Q 19/00

(54) **CATERPILLAR FUNGUS EXTRACT, PREPARATION METHOD AND USE THEREOF**

(30) Priority: 31.12.2024 CN 202411997313
(71) Applicant: Infinitus (China) Company Ltd., Jiangmen, Guangdong 529156 (CN)
(72) Inventor: TAI, Meiling, Jiangmen, 529156 (CN); LI, Wanzhao, Jiangmen, 529156 (CN)
(74) Representative: Kehl, Ascherl, Liebhoff & Ettmayr Patentanwälte Partnerschaft mbB

(57) **Abstract**

Disclosed are an extract of Caterpillar Fungus, and a method for preparing the same and use thereof. The extract of caterpillar fungus is obtained by extracting caterpillar fungus with a polyol aqueous solution and EDTA-4Na, and filtering and removing impurities. The extract is free of DNA, and can repair cellular DNA damage caused by ultraviolet light, inhibit the production of cyclobutane pyrimidine dimers, thereby reducing or treating skin photoaging. Furthermore, the use of stabilizers and preservatives can be reduced or avoided in the cosmetics prepared based on the extract, which meets the general requirements of consumers for the safety of cosmetics and improves the compliance.

## Description

### TECHNICAL FIELD

The present disclosure relates to the technical field of extraction of natural active substances, and in particular, to an extract of Caterpillar Fungus, and a method for preparing the same and use thereof.

### BACKGROUND

Sunlight consists of gamma ray, X-ray, ultraviolet light, visible ray and infrared light. Among these, ultraviolet light has a wavelength between 200 nm and 400 nm, visible light has a wavelength between 400 nm and 760 nm, and infrared ray has a wavelength above 760 nm. The ultraviolet light irradiating the surface of the Earth can be divided into: UVC region (200 nm-290 nm), UVB region (290 nm-320 nm) and UVA region (320 nm-400 nm). Ultraviolet light has a direct impact on skin cell DNA, resulting in the generation of cyclobutane pyrimidine dimers (CPDs), also known as Dark CPDs, in the skin cells. When UV-induced reactive oxygen species trigger the chemical excitation of melanin, the energy will be transferred to DNA (which is referred as Ultraviolet Photon Transfer), so as to induce the production of the CPDs. This phenomenon usually continues to occur 3-4 hours or even longer after the exposure to ultraviolet irradiation; that is, even after stopping exposure to solar radiation or ultraviolet irradiation, the damage to the skin cells, especially DNA damage, will continue to occur.

Thioredoxin-interacting protein (TXNIP) is a type of thioredoxin-binding protein that can mediate oxidative stress, inhibit cell proliferation, and induce cell apoptosis by inhibiting the function of the thioredoxin system. In addition, TXNIP is also closely involved in the DNA methylation process in blood glucose control.

Chinese Caterpillar Fungus, or more simply Caterpillar Fungus or Aweto, refers to the stroma of Cordyceps Genus fungus, such as *Cordyceps sinensis* (BerK.) Sacc., parasitic on the larva of the insect of Hepialidae family, or a complex of the Cordyceps Genus fungus with a body of the larva. Studies have shown that caterpillar fungus extract processed by small molecular ribonucleic acid (RNA) extraction technology can effectively inhibit the production of cyclobutane pyrimidine dimers and repair the damage to skin cell DNA caused by thioredoxin-interacting proteins. However, the small molecular RNA extraction technology for caterpillar fungus in the existing technology cannot meet the actual needs. Although the existing plant small RNA extraction methods, such as those disclosed in CN108291222A, solve the technical difficulty of stably extracting small RNA in large-scale production, they require the introduction of stabilizers and preservative systems into the final aqueous extract for the best use effect. However, the use of these additives is not actually in line with the current additive-free concept, and the consumers have increasingly higher requirements on the stability and safety of the product itself.

Therefore, it is of substantial significance to develop a caterpillar fungus extract containing small molecular RNA without introducing a preservative system, thereby opening a novel solution for the deep development of caterpillar fungus.

### SUMMARY

The present disclosure aims to solve at least one of the technical problems existing in the existing technology. In view of this, an object of the present disclosure is to provide an extract of Caterpillar Fungus, and a method for preparing the same and use thereof. The present disclosure realizes, for the first time, the preparation of an extract of caterpillar fungus containing small molecular RNA. The prepared extract of caterpillar fungus is rich in small molecular RNA, can repair cellular DNA damage caused by ultraviolet light and inhibit the production of cyclobutane pyrimidine dimers, thereby reducing or treating skin photoaging. Furthermore, the stabilizers and preservatives can be reduced or avoided in the cosmetics prepared based on the extract, which meets the general requirements of consumers for the safety of cosmetics and improves the compliance.

According to a first aspect of the present disclosure, provided is a method for preparing an extract of caterpillar fungus, including the following steps:
crushing the caterpillar fungus, adding a polyol aqueous solution and then EDTA-4Na for extraction, filtering and removing impurities, adjusting a pH to 6-6.5, and optionally filtering again, to obtain an extract of caterpillar fungus;
wherein, the filtering is conducted with a pore size of 0.2-0.3 µm, and the extraction is conducted at a temperature of 50-80°C for a period of 30-90 min.

In some embodiments of the present disclosure, the extraction is conducted at a temperature of 60-80°C.

In some embodiments of the present disclosure, the caterpillar fungus includes any pharmaceutically acceptable part of caterpillar fungus.

In some embodiments of the present disclosure, the caterpillar fungus is a complete strain of caterpillar fungus, that is, a complex of a stroma of Cordyceps Genus fungus with a body of an insect. In some embodiments, the Cordyceps Genus fungus includes *Cordyceps sinensis* (BerK.) Sacc. In some embodiments, the insect includes an insect of Hepialidae family.

In some embodiments of the present disclosure, the polyol includes any one of ethylene glycol, propylene glycol, glycerol, trimethylolethane, and pentaerythritol.

In some embodiments of the present disclosure, the polyol is selected from the group consisting of ethylene glycol, propylene glycol, and glycerol;

In some embodiments of the present disclosure, the polyol is propylene glycol.

In some embodiments of the present disclosure, in the polyol aqueous solution, a ratio by weight of polyol to water is (4-4.5): (5.5-6).

In some embodiments of the present disclosure, in the polyol aqueous solution, the ratio by weight of polyol to water is 4:6.

In some embodiments of the present disclosure, an amount of the caterpillar fungus is 0.5-2% by weight of the polyol aqueous solution.

In some embodiments of the present disclosure, the amount of the caterpillar fungus is 0.5-1% by weight of the polyol aqueous solution.

In some embodiments of the present disclosure, the amount of the caterpillar fungus is 1% by weight of the polyol aqueous solution.

In some embodiments of the present disclosure, during the extraction, a pH is maintained between 10.5 and 11.

In some embodiments of the present disclosure, the extraction conducted at 80°C for 60 minutes.

In some embodiments of the present disclosure, the filtering and removing impurities includes: removing insoluble solids and high molecular weight nucleic acids.

In some embodiments of the present disclosure, the filtering and removing impurities includes: removing high molecular weight RNA and all DNA.

In some embodiments of the present disclosure, the filtering and removing impurities is conducted by filtering sequentially using filters with decreasing porosity.

In some embodiments of the present disclosure, the porosity of the filters decreases from 20-50 µm to 7-20 µm.

According to a second aspect of the present disclosure, provided is an extract of caterpillar fungus prepared by the preparation method described in the above aspect.

In some embodiments of the present disclosure, the extract of caterpillar fungus contains ribonucleic acid (RNA) and does not contain deoxyribonucleic acid (DNA).

In some embodiments of the present disclosure, the ribonucleic acid is a ribonucleic acid with a molecular weight less than or equal to 150 Daltons.

In some embodiments of the present disclosure, the ribonucleic acid has a content in the extract of caterpillar fungus content of greater than or equal to 5 mg/kg.

In some embodiments of the present disclosure, the extract of caterpillar fungus further includes at least one of protein fragments, saccharides, amino acids, phenolic compounds, organic acids, and vitamins.

In some embodiments of the present disclosure, the extract of caterpillar fungus further includes at least one of the following components in the content ranges thereof: 0.5-1.5 g/kg protein fragments, 0.5-1.5 g/kg saccharides, 0.2-1.0 g/kg amino acids, 50-150 mg/kg phenolic compounds, 0.05-0.25 g/kg organic acids, and 0.1-0.5 mg/kg vitamins.

In some embodiments of the present disclosure, a dry matter amount of the extract of caterpillar fungus is 5-15 g/kg.

In some embodiments of the present disclosure, the extract of caterpillar fungus includes: 0.5-1.5 g/kg protein fragments, 0.5-1.5 g/kg saccharides, 0.2-1.0 g/kg amino acids, 50-150 mg/kg phenolic compounds, 0.05-0.25 g/kg organic acids, 0.1-0.5 mg/kg vitamins, 5-20 mg/kg ribonucleic acid with a molecular weight less than or equal to 150 Daltons.

According to a third aspect of the present disclosure, provided is a composition containing the extract of caterpillar fungus described in the above aspects.

In some embodiments of the present disclosure, the composition contains 0.1-5% by weight of the extract of caterpillar fungus.

In some embodiments of the present disclosure, the composition contains 2-5% by weight of the extract of caterpillar fungus.

In some embodiments of the present disclosure, the composition contains 5% by weight of the extract of caterpillar fungus.

In some embodiments of the present disclosure, the composition further contains an auxiliary agent.

In some embodiments of the present disclosure, the composition does not contain a preservative or a stabilizer.

In some embodiments of the present disclosure, the auxiliary agent is selected from a group consisting of a solvent, a humectant, a pH regulator, an antioxidant, a defoaming agent, an emulsifier, a colorant and a skin conditioning agent.

In some embodiments of the present disclosure, the composition may be in a form of an aqueous solution, a hydroalcoholic solution or an oil solution, or may be an emulsion or multiple emulsion in a form of oil-in-water or water-in-oil, or an aqueous gel, etc. These compositions may be fluid and in a form of a cream, a lotion, an emulsion, a serum, a gel, a paste or a foam, or may be in a solid form, for example a stick solid, or in an aerosol form, for application to the skin.

According to a fourth aspect of the present disclosure, provided is use of the extract of caterpillar fungus or the composition described in the above aspects in the preparation of a medicine or a cosmetic.

In some embodiments of the present disclosure, the medicine or cosmetic has at least one of the following functions:
(1) resisting ultraviolet radiation;
(2) promoting skin repair; or
(3) preventing or treating skin damage.

According to a fifth aspect of the present disclosure, provided is a method for resisting ultraviolet radiation, including: applying an effective amount of the extract of caterpillar fungus or the composition described in the above aspects to the skin of a subject.

As used in the present disclosure, the term "effective amount" refers to an amount sufficient to obtain, or at least partially obtain, the desired effect. For example, a prophylactically effective amount refers to an amount sufficient to prevent, arrest or delay the onset of a disease. For example, a therapeutically effective amount is an amount sufficient to cure, alleviate, or prevent further progression or progression of a disease.

As used in the present disclosure, the term "subject" includes non-human animal and human, preferably human. According to the present disclosure, the administered amount may be adjusted based on the selection of the subject, for example, a dose may be adjusted according to the conventional dose conversion relationship among different species in the art.

According to a sixth aspect of the present disclosure, provided is a method for promoting skin repair, including: applying an effective amount of the extract of caterpillar fungus or the composition described in the above aspects to the skin of a subject in need.

In some embodiments of the present disclosure, the skin has cell damage or DNA damage.

In some embodiments of the present disclosure, the damage is caused by ultraviolet radiation.

According to a seventh aspect of the present disclosure, provided is a method for preventing or treating skin damage, including: applying an effective amount of the extract of caterpillar fungus or the composition described in the above aspects to the skin of a subject in need.

In some embodiments of the present disclosure, the skin damage is caused by ultraviolet radiation.

The present disclosure achieves the following beneficial effects:
1. The present disclosure provides a method for preparing an extract of caterpillar fungus. Compared with the extract obtained by the conventional extraction method, the extract of caterpillar fungus obtained by the method is richer in small molecular RNA with a maximum length of 150 nucleotides, and has better effects of physiological acceptability and UV resistance. By applying a composition containing the extract, cellular DNA damage caused by ultraviolet light can be repaired, the production of cyclobutane pyrimidine dimers can be inhibited, thereby reducing or treating skin photoaging.
2. In the present disclosure, an extract of caterpillar fungus rich in small molecular RNA with a maximum length of 150 nucleotides is obtained for the first time, and used in the cosmetics, which not only provides an anti-ultraviolet effect, but also avoids the use of stabilizers and preservatives, meets the general requirements of the consumers for the safety of cosmetics and improves the compliance.

### BRIEF DESCRIPTION OF DRAWINGS

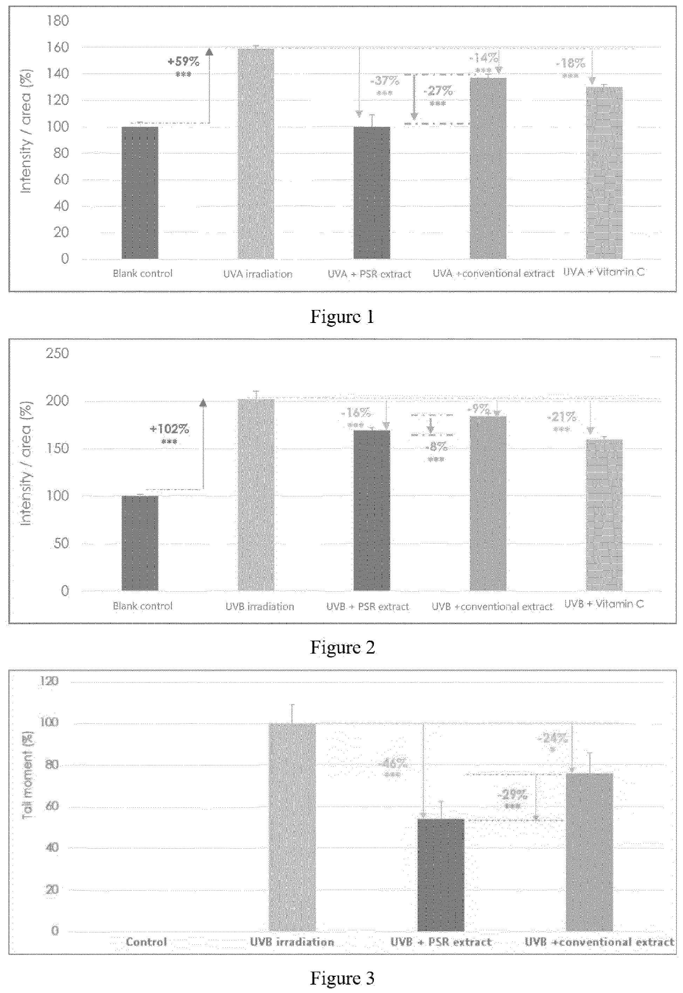

Figure 1 shows the effect of an extract of caterpillar fungus according to an embodiment of the present disclosure on the TXNIP content of ex vivo skin tissue after ultraviolet irradiation, where the statistical method is non-cross-sectional statistical analysis (mean ± sem; n=6), and *** indicates a high significance verified by Student t test.
Figure 2 shows the effect of an extract of caterpillar fungus according to an embodiment of the present disclosure on the CPD production of ex vivo skin tissue after ultraviolet irradiation, where the statistical method is non-cross-sectional statistical analysis (mean ± sem; n=6), *** indicates a high significance verified by Student t test, and * indicates a significance.
Figure 3 shows the effect of an extract of caterpillar fungus according to an embodiment of the present disclosure of anti-ultraviolet damage of human skin keratinocytes, where the statistical method is non-cross-sectional statistical analysis (mean ± sem; n=6), *** indicates a high significance verified by Student t test, and * indicates a significance.

### DETAILED DESCRIPTION

The present disclosure is further described in detail below in combination with particular embodiments. Unless otherwise specified, the raw materials, reagents or devices used in the examples and comparative examples can be obtained from conventional commercial sources, or can be obtained by methods in the existing technology. Unless otherwise specified, the experimental or test methods are conventional in the art.

### Example 1 - A preservative-free extract of Caterpillar Fungus (Cordyceps sinensis)

In this example, a method for preparing a small RNA-rich, preservative-free *Cordyceps sinensis* extract was provided, which included the following steps.
(1) A complete plant of *Cordyceps sinensis* was washed thoroughly and then dried, then ground into a powder with uniform particles, and then added with a mixed solution of distilled water and propylene glycol in a ratio of 60:40 to mix, wherein the added amount of *Cordyceps sinensis* powder was 1% by weight of the mixed solution of distilled water and propylene glycol. In this example, 10 grams of *Cordyceps sinensis* powder was added to the mixed solution obtained by mixing 600 grams of distilled water and 400 grams of propylene glycol. Then EDTA-4Na was added to the mixture with a final concentration of 10 mM and stirred thoroughly for extraction. During this process, the pH value should be controlled between 10.5 and 11 to achieve efficient extraction of low molecular weight *Cordyceps sinensis* RNA. The extraction was carried out at 80 °C for 1 hour.
(2) After the extraction, the extract was continuously filtered using filters with decreasing porosity (from 20-50 µm to 7-20 µm) to remove insoluble solids and high molecular weight DNA, to obtain a clear aqueous extract.
(3) The pH value of the clear aqueous extract was detected and adjusted to between 6 and 6.5 using citric acid, so as to retain small molecular RNA in the extract. It should be noted that further acidification or too low pH value would cause precipitation of the small molecular RNA in the extract, affecting the yield.
(4) The extract was further aseptically filtered using a filter with a porosity of 0.2-0.3 µm to obtain a final product.

### Example 2 - A preservative-free extract of Caterpillar Fungus (Cordyceps sinensis)

In this example, a method for preparing a small RNA-rich, preservative-free *Cordyceps sinensis* extract was provided, which included the following steps:
(1) A complete plant of *Cordyceps sinensis* was washed thoroughly and then dried, then ground into a powder with uniform particles, and then added with a mixed solution of distilled water and propylene glycol in a ratio of 60:40 to mix, wherein the added amount of *Cordyceps sinensis* powder was 1% by weight of the mixed solution of distilled water and propylene glycol. In this example, 10 grams of *Cordyceps sinensis* powder was added to the mixed solution obtained by mixing 600 grams of distilled water and 400 grams of propylene glycol. Then EDTA-4Na was added to the mixture with a final concentration of 10 mM and stirred thoroughly for extraction. During this process, the pH value should be controlled between 10.5 and 11 to achieve efficient extraction of low molecular weight *Cordyceps sinensis* RNA. The extraction was carried out at 60 °C for 45 minutes.
(2) After the extraction, the extract was continuously filtered using filters with decreasing porosity (from 20-50 µm to 7-20 µm) to remove insoluble solids and high molecular weight DNA to obtain a clear aqueous extract.
(3) The pH value of the clear aqueous extract was detected and adjusted to between 6 and 6.5 using citric acid, so as to retain small molecular RNA in the extract. It should be noted that further acidification or too low pH value would cause precipitation of the small molecular RNA in the extract, affecting the yield.
(4) The extract was further aseptically filtered using a filter with a porosity of 0.2-0.3 µm to obtain a final product.

### Example 3 - A preservative-free extract of Caterpillar Fungus (Cordyceps sinensis)

In this example, a method for preparing a small RNA-rich, preservative-free *Cordyceps sinensis* extract was provided, which included the following steps:
(1) A complete plant of *Cordyceps sinensis* was washed thoroughly and then dried, then ground into a powder with uniform particles, and then added with a mixed solution of distilled water and propylene glycol in a ratio of 60:40 to mix, wherein the added amount of *Cordyceps sinensis* powder was 1% by weight of the mixed solution of distilled water and propylene glycol. In this example, 10 grams of *Cordyceps sinensis* powder was added to the mixed solution obtained by mixing 600 grams of distilled water and 400 grams of propylene glycol. Then EDTA-4Na was added to the mixture with a final concentration of 10 mM and stirred thoroughly for extraction. During this process, the pH value should be controlled between 10.5 and 11 to achieve efficient extraction of low molecular weight *Cordyceps sinensis* RNA. The extraction was carried out at 70 °C for 30 minutes.
(2) After the extraction, the extract was continuously filtered using filters with decreasing porosity (from 20-50 µm to 7-20 µm) to remove insoluble solids and high molecular weight DNA to obtain a clear aqueous extract.
(3) The pH value of the clear aqueous extract was detected and adjusted to between 6 and 6.5 using citric acid, so as to retain small molecular RNA in the extract. It should be noted that further acidification or too low pH value would cause precipitation of the small molecular RNA in the extract, affecting the yield.
(4) The extract was further aseptically filtered using a filter with a porosity of 0.2-0.3 µm to obtain a final product.

### Example 4 - Stability and preservative effect of the extract of Caterpillar Fungus (Cordyceps sinensis)

In this example, the inventors designed a stability test (at room temperature, 0-4°C and 40°C) and a preservative test (a 28-day complete preservative efficacy test) to determine the stability and preservative effect of the extracts of *Cordyceps sinensis* obtained according to the method in the above examples and using different ratios of water to polyol.

The experiments were carried out as follows.

### (1) Stability test

The inventors tested the stability under different environments of the extracts of *Cordyceps sinensis* obtained according to the method in Example 1 and using water and polyols at different ratios as extraction media. The specific test method was as follows: the extracts of *Cordyceps sinensis* obtained using water and polyols at different ratios as extraction media were obtained by referring to the method in Example 1; the amount of dry matter of the extracts was detected by the loss on drying method (the extracts were dried in a ventilated oven at 105 ± 2°C), and the RNA content was detected by a microanalyzer (Agilent 2100 Bio-Analyzer and Nano RNA Chip Kit); and then the extracts were sealed and stored at room temperature (RT), 0-4°C and 40°C for 3 months separately to observe changes of the extracts of *Cordyceps sinensis.*

The results were shown in Table 1 below.

**Table 1. Stability of the extracts of Cordyceps sinensis obtained using water and polyols at different ratios as extraction media**

| Sample | Storage Condition | | | Dry Matter Amount (g/kg) | RNA Content (ppm) |
|---|---|---|---|---|---|
| | RT | 4°C | 40°C | | |
| Extract (water: propylene glycol =90:10) | Slightly crystallized | Crystallized | Stable without change | 7 | 10 |
| Extract (water: propylene glycol =80:20) | Stable without change | Crystallized | Stable without change | 7.3 | 10 |
| Extract (water: propylene glycol =70:30) | Stable without change | Slightly crystallized | Stable without change | 7.3 | 11 |
| Extract (water: propylene glycol =65:35) | Stable without change | Slightly crystallized | Stable without change | 7.5 | 11 |
| Extract (water: propylene glycol =60:40) | Stable without change | Stable without change | Stable without change | 7.5 | 11 |
| Extract (water: propylene glycol =55:45) | Stable without change | Stable without change | Stable without change | 7.5 | 11 |

### (2) Preservative effect test

The microorganisms shown in the following Table 2 were cultured with culture mediums, and then inoculated into plates containing the extracts of *Cordyceps sinensis* obtained using water and polyols at different ratios as extraction media, and incubated under normal culture condition. The microbial count was performed at 2, 7, 14, 21, and 28 days after inoculation separately. After the total number of colonies was calculated on Day 21, inoculation was performed again to complete the standard preservative efficacy test (PET). Four bacterial strains were mixed in equal amounts and inoculated for separate experiments, and the yeast and mold strains were mixed in equal amounts and inoculated for separate experiments.

**Table 2. Information of the microorganisms used in preservative effect test**

| Strains | | Sources | Inoculated Dose |
|---|---|---|---|
| Bacteria | *Psedudomonas aeruginosa* ATCC 9027 | ATCC | 1.00E+06 |
| | *Staphylococcus aureus* ATCC 6538 | ATCC | |
| | *Burkholderia cepacia* ATCC 25416 | ATCC | |
| | *Escherichia coli* ATCC 8739 | ATCC | |
| Yeast | *Candida albicans* ATCC 10231 | ATCC | 1.00E+05 |
| Mold | *Aspergillus niger* ATCC 16404 | ATCC | 1.00E+05 |

The results were shown in Table 3.

**Table 3. Preservative effect of the extracts of Cordyceps sinensis obtained using water and polyols at different ratios as extraction media**

| Sample | Microorganisms | Colony Count (cfu/mL) | | | | |
|---|---|---|---|---|---|---|
| | | 2d | 7d | 14d | 21d | 28d |
| Extract (water: propylene glycol =90:10) | Bacteria | 1.2×10⁴ | 3.4×10³ | N/A | N/A | N/A |
| | Mold | 7.4x 10³ | 4.7×10³ | N/A | N/A | N/A |
| | Yeast | 5.8×10⁴ | 2.9×10³ | N/A | N/A | N/A |
| Extract (water: propylene glycol =80:20) | Bacteria | 7.4×10³ | 2.1×10² | N/A | N/A | N/A |
| | Mold | 5.3×10³ | 4.9×10² | N/A | N/A | N/A |
| | Yeast | 9.4×10³ | 1.7×10³ | N/A | N/A | N/A |
| Extract (water: propylene glycol =70:30) | Bacteria | 1.5×10² | 0.4×10² | <10 | <10 | 1.9×10² |
| | Mold | 7.8×10² | <10 | <10 | <10 | 2.3×10² |
| | Yeast | 3.9×10⁴ | 1.0×10¹ | <10 | <10 | 4.2×10² |
| Extract (water: propylene glycol =65:35) | Bacteria | 5.6×10² | <10 | <10 | <10 | <10 |
| | Mold | 9.4×10³ | <10 | <10 | <10 | <10 |
| | Yeast | 1.2×10⁴ | <10 | <10 | <10 | <10 |
| Extract (water: propylene glycol =60:40) | Bacteria | 2.8×10² | <10 | <10 | <10 | <10 |
| | Mold | 3.2×10³ | <10 | <10 | <10 | <10 |
| | Yeast | 2.9×10³ | <10 | <10 | <10 | <10 |
| Extract (water: propylene glycol =55:45) | Bacteria | 1.3×10² | <10 | <10 | <10 | <10 |
| | Mold | 4.6×10³ | <10 | <10 | <10 | <10 |
| | Yeast | 1.3×10⁴ | <10 | <10 | <10 | <10 |

In the above table, N/A indicated "unable to count for overabundance".

The results in the above table showed that, the extracts of *Cordyceps sinensis* obtained using a mixed solution of 55%-60% water and 40%-45% propylene glycol as the extraction medium had better stability and preservative effect than those obtained in the other ratio ranges. Taking account of the cost, the extract of *Cordyceps sinensis* obtained using a mixed solution of 60% water and 40% propylene glycol as the extraction medium had the best comprehensive effect.

### Example 5 - Component analysis of the extract of Caterpillar Fungus (Cordyceps sinensis)

The component analysis was performed on the extracts of *Cordyceps sinensis* obtained in Examples 1 to 3. The amount of dry matter was detected by the loss on drying method (similar to that in Example 4), the RNA content was detected by a microanalyzer (similar to that Example 4), and the content of other active substances was detected by HPLC. The results were as follows.

The extracts of *Cordyceps sinensis* obtained in Examples 1 to 3 were slightly yellow or yellow in color and contained 5-15 g/kg of dry matter, including 0.5-1.5 g/kg of protein fragments, 0.5-1.5 g/kg of saccharides, 0.2-1.0 g/kg of amino acids, 50-150 mg/kg of phenolic compounds, 0.05-0.25 g/kg of organic acids, 0.1-0.5 mg/kg of vitamins, and 5-20 mg/kg of small molecular RNA with a maximum nucleotide length of 150.

The extract of *Cordyceps sinensis* obtained in Example 1 using a mixed solution of 60% water and 40% propylene glycol as the extraction medium contained 7.5 g/kg dry matter, including 0.71 g/kg protein fragments (in molar mass of 205-1,330 g/mol), 0.74 g/kg saccharides (mainly mannose, also referred as cordycepic acid), 0.5 g/kg amino acid (mainly glutamate, arginine, alanine and proline), 74 mg/kg phenolic compounds, 0.12 g/kg organic acids (mainly lactic acid and malic acid), 0.3 mg/kg vitamins (mainly B3-b-nicotinic acid and B3-a-nicotinamide), and 11 mg/kg small molecular RNA with a maximum nucleotide length of 150. Moreover, the gel electrophoresis analysis showed that the molecular weight of RNA in the extract of *Cordyceps sinensis* was less than or equal to 150 nucleotides in length, and the extract did not contain deoxyribonucleic acid (DNA) at all.

The extracts of *Cordyceps sinensis* obtained in Examples 2 and 3 were also detected as above, and the results were similar to those of the extract of *Cordyceps sinensis* obtained in Example 1.

### Example 6 - Differences in components between the extract of Cordyceps sinensis in the present disclosure and the extract obtained by conventional methods

The inventors extracted an extract of *Cordyceps sinensis* using the same amounts of *Cordyceps sinensis,* distilled water and propylene glycol as in Example 1 (1% *Cordyceps sinensis, 60%* distilled water, and 40% propylene glycol) according to a conventional solvent extraction method (using the same solvent, the same operating temperature, and the same amount of plant feed as in Example 1, but without adding EDTA-4Na), and performed component analysis according to the method in the above example and compared the components with those of the extract of *Cordyceps sinensis* in Example 1. The results were shown in Table 4.

**Table 4. Comparison of components of the extracts of Cordyceps sinensis obtained by different methods**

| | Extract of *Cordyceps sinensis* of the present disclosure | Extract of *Cordyceps sinensis* obtained by conventional method |
|---|---|---|
| Dry matter (g/kg) | 7.5 | 0.8 |
| Total proteins (g/kg) | 0.71 | 0.4 |
| Total saccharides (g/kg) | 0.74 | 0.41 |
| Total amino acids (g/kg) | 0.5 | 0.24 |
| Total phenolic compounds (g/kg) | 0.074 | 0.026 |
| Organic acids (g/kg) | 0.12 | 0.045 |
| Vitamins (g/kg) | 0.0003 | 0.0001 |
| Small RNA (g/kg) | 0.011 | 0 |

### Example 7 Anti-ultraviolet damage effect of the extract of Cordyceps sinensis in the present disclosure

The extract of *Cordyceps sinensis* in the examples of the present disclosure was generally mixed in an amount of 0.1-5% of the total weight of the composition with other components to prepare a cosmetic composition for practical use. Preferably, the amount of the extract of *Cordyceps sinensis* was added in an amount of 0.5-2% of the total weight of the composition. The cosmetic composition containing the extract of *Cordyceps sinensis* in the examples of the present disclosure may be in a form of an aqueous solution, a hydroalcoholic solution or an oil solution, or may be an emulsion or multiple emulsion in a form of oil-in-water or water-in-oil, or an aqueous gel, etc. These compositions may be fluid and in a form of a cream, a lotion, an emulsion, a serum, a gel, a paste or a foam, or may be in a solid form, for example a stick solid, or in an aerosol form, for application to the skin. In this example, the inventors dissolved the extracts of *Cordyceps sinensis* in the above examples in deionized water at an amount of 0.5% by weight to simulate a cosmetic composition, and verified its repair effect on damage caused by ultraviolet light.

The specific experimental method was as follows.

The ex vitro skin tissues were irradiated by UVA with an intensity of 5 J/cm² to increase the expression of TXNIP, thereby simulating the production of endotoxic substance thioredoxin-interacting protein (TXNIP) in normal skin as a result of exposure to ultraviolet light (mainly UVA), and the skin damage. Then an aqueous solution containing 0.5% by weight of the aforesaid extract of *Cordyceps sinensis* or the extract of *Cordyceps sinensis* obtained by the aforesaid conventional extraction method was applied to the skin tissues respectively, and the skin tissues were incubated for 2 days. The culture medium containing the corresponding extracts was respectively replaced twice a day. One day after culture (24 hours), radiation was performed. Rabbit monoclonal anti-TXNIP antibody (dilution 1:200, Abcam) was used to determine the changes in TXNIP content by ELISA assay. A 200 µg/mL vitamin C aqueous solution was used as a positive control, and the group without UVA irradiation was used as a blank control.

The results were shown in Figure 1.

The results showed that, the TXNIP content in the group containing 0.5% of the extract of *Cordyceps sinensis* of the present disclosure (abbreviated as: PSR extract) was reduced by 37% compared with that in the UVA irradiation group, and reduced by 27% compared with the group containing 0.5% of the extract of *Cordyceps sinensis* obtained by the conventional method (abbreviated as: conventional extract). Therefore, it indicated that the extract of *Cordyceps sinensis* prepared by the extraction process in the examples of the present disclosure had a better anti-ultraviolet radiation effect than the extract of *Cordyceps sinensis* obtained by the conventional method.

In addition, the inventors further detected the changes in cyclobutane pyrimidine dimer (CPD) in ex vivo skin tissues that could be used for characterizing the degree of DNA damage in living cells under ultraviolet radiation pressure, so as to determine the effect of the extract of *Cordyceps sinensis* on damage repair.

The specific experimental method was as follows.

The ex vitro skin tissues were irradiated by UVB with an intensity of 200 mJ/cm² to increase the expression of cyclobutane pyrimidine dimers in cell DNA, then an aqueous solution containing 0.5% by weight of the aforesaid extract of *Cordyceps sinensis* or the extract of *Cordyceps sinensis* obtained by the aforesaid conventional extraction method was applied to the skin tissues respectively, and the skin tissues were incubated for 2 days. The culture medium containing the corresponding extracts was respectively replaced twice a day. One day after culture (24 hours), radiation was performed. Mouse polyclonal anti-CPD antibody (dilution 1:250, Euromedex) was used to determine the changes in CPD expression levels by ELISA assay. A 200 µg/mL vitamin C aqueous solution was used as a positive control, and the group without UVB irradiation was used as a blank control.

The results were shown in Figure 2.

The results showed that, the CPD expression level in the group containing 0.5% of the extract of *Cordyceps sinensis* of the present disclosure (abbreviated as: PSR extract) was reduced by 16% compared with that in the UVB irradiation group, and reduced by 8% compared with the group containing 0.5% of the extract of *Cordyceps sinensis* obtained by the conventional method (abbreviated as: conventional extract). Therefore, it indicated that the extract of *Cordyceps sinensis* prepared by the extraction process in the examples of the present disclosure had a better repair effect on the UVB-induced persistent skin damage than the extract of *Cordyceps sinensis* obtained by the conventional method.

In order to further verify the anti-ultraviolet damage effect of the extract of *Cordyceps sinensis* in the present disclosure, the inventors conducted a DNA UV damage comet assay using commercially available human skin keratinocyte as the experimental object.

The specific experimental method was as follows.

Human skin keratinocytes were cultured for 2 days in a culture medium containing the aqueous solution containing 0.5% of the extract of *Cordyceps sinensis* of the present disclosure or 0.5% of the *Cordyceps sinensis* extract obtained by the conventional method. The culture medium was replaced twice a day. At the 24th hour of culture, UVB irradiation with an intensity of 60 mJ/cm² was performed. The cells without UVB irradiation and the cells that were irradiated by UVB but not treated with any extract were set as controls.

The results were shown in Figure 3.

The results showed that, the tail moment in the group containing 0.5% of the extract of *Cordyceps sinensis* of the present disclosure (abbreviated as: PSR extract) was reduced by 46% compared with that in the UVB irradiation group, and reduced by 24% compared with the group containing 0.5% of the extract of *Cordyceps sinensis* obtained by the conventional method (abbreviated as: conventional extract), which was significantly different. Therefore, it indicated that the extract of *Cordyceps sinensis* prepared by the extraction process in the examples of the present disclosure had a better repair effect on the UVB-induced persistent skin photodamage than the extract of *Cordyceps sinensis* obtained by the conventional method.

The above examples are preferred embodiments of the present disclosure, but the embodiments of the present disclosure are not limited to the above examples. Any other changes, modifications, substitutions, combinations, and simplifications, and the like made without departing from the technical solutions of the present disclosure should be considered as equivalent substitutions, and should fall within the protection scope of the present disclosure.

## Claims

1. A method for preparing an extract of caterpillar fungus, comprising the following steps:
crushing the caterpillar fungus, adding a polyol aqueous solution and then EDTA-4Na for extraction, filtering and removing impurities, adjusting a pH to 6-6.5, and optionally filtering again, obtain the extract of caterpillar fungus;
wherein, the filtering is conducted with a pore size of 0.2-0.3 µm, and the extraction onducted at a temperature of 50-80 °C for a period of 30-90 min.

2. The method according to claim 1, wherein the polyol comprises any one of ethylene glycol, propylene glycol, glycerol, trimethylolethane, and pentaerythritol.

3. The method according to claim 1, wherein, in the polyol aqueous solution, a ratio by weight of polyol to water is (4-4.5): (5.5-6).

4. The method according to claim 1, wherein an amount of the caterpillar fungus is 0.5-2% by weight of the polyol aqueous solution.

5. The method according to claim 1, wherein, during the extraction, a pH is maintained between 10.5 and 11.

6. An extract of caterpillar fungus prepared by the method of any one of claims 1 to 5, wherein the extract of caterpillar fungus comprises ribonucleic acid and does not contain deoxyribonucleic acid.

7. The extract of caterpillar fungus according to claim 6, wherein the ribonucleic acid is a ribonucleic acid with a molecular weight less than or equal to 150 Daltons.

8. The extract of caterpillar fungus according to claim 6, wherein, the ribonucleic acid has a content in the extract of caterpillar fungus content of greater than or equal to 5 mg/kg.

9. The extract of caterpillar fungus according to claim 6, wherein the extract of caterpillar fungus further comprises at least one of the following components in the content ranges thereof: 0.5-1.5 g/kg protein fragments, 0.5-1.5 g/kg saccharides, 0.2-1.0 g/kg amino acids, 50-150 mg/kg phenolic compounds, 0.05-0.25 g/kg organic acids, and 0.1-0.5 mg/kg vitamins.

10. A composition comprising the extract of caterpillar fungus of any one of claims 6 to 9.

11. The composition according to claim 10, wherein the composition comprises 0.1-5% by weight of the extract of caterpillar fungus.

12. The composition according to claim 10, wherein the composition further comprises an auxiliary agent; and the auxiliary agent does not comprise a preservative or a stabilizer.

13. The composition according to claim 12, wherein the auxiliary agent is selected from at least one of a group consisting of a solvent, a humectant, a pH regulator, an antioxidant, a defoaming agent, an emulsifier, a colorant and a skin conditioning agent.

14. The extract of caterpillar fungus of any one of claims 6 to 9 or the composition of any one of claims 10 to 13 for use in the preparation of a medicine or a cosmetic.

15. The extract of caterpillar fungus of any one of claims 6 to 9 or the composition of any one of claims 10 to 13 for use in at least one of:
(1) resisting ultraviolet radiation;
(2) promoting skin repair; or
(3) preventing or treating skin damage.
